# EUROPEAN PATENT APPLICATION

(11) **EP 2 878 289 A1**
(43) Date of publication of application: **03.06.2015**
(21) Application number: 13195163.4
(22) Date of filing: 29.11.2013
(51) Int. Cl.: A61F 13/42

(54) **An incontinence garment, a soiling alert transmitter, a soiling alert system, and methods for detecting soiling of an incontinence garment**

(71) Applicant: NXP B.V., 5656 AG Eindhoven (NL)
(72) Inventor: De Troch, Stefan, Redhill, Surrey RH1 1SH (GB); Schaafsma, Siebren, Redhill, Surrey RH1 1SH (GB); Brongersma, Sywert, Redhill, surrey RH1 1SH (GB)
(74) Representative: Hardingham, Christopher Mark

(57) **Abstract**

A soiling alert transmitter is disclosed for integration into an incontinence garment having an interior region and inward-facing and outward-facing surface regions, the transmitter comprising: a sensor configured to sense humidity changes in the inward-facing surface region of the incontinence garment; a processing unit configured to detect whether a sensed change is a transient change or a permanent change; and a magnetic induction radio configured to transmit an alert signal in response to the detection of a transient change. Incontinence garments having such soiling alert transmitters integrated therein, soiling alert systems, and methods for detecting soiling of such an incontinence garment are also disclosed.

## Description

### Field of the Invention

This invention relates to incontinence garments, and to methods, detectors and systems for detecting soiling of incontinence garments.

### Background of the Invention

Due to ageing populations in many regions of the world, use of incontinence garments such as diapers (also known as "nappies") is widespread not only for infants and babies, but also for increasingly for patients towards the end of life and other adults.

Changing of incontinence garments such as diapers when they become saturated, or fouled due to excrement - stools or faeces, is a basic aspect of health care for incontinent patients. It is important to know when it is necessary to change the diaper: on the one hand, changing diapers more often than is necessary is expensive and wasteful of diaper materials and care-worker effort, and overly intrusive and disruptive; on the other hand if a diaper is not changed at an appropriate time, there is a significant risk of skin irritation which may lead to the requirement for further medical intervention and treatment which may take several weeks, and be more disruptive and expensive.

Odour or touch may be used to detect the fouling of a diaper; in all but the worst cases this may require close inspection: whereas with infants, this may be acceptable, with adult and elderly patients it may be considered to be intrusive. It would therefore be desirable to provide automated systems for providing an appropriate alert when a diaper is required to be changed.

It is known to provide monitoring systems for the occurrence of wetness within a diaper. For example United States patent application publication number US2008/0262453 discloses a remote monitoring diaper system, kits and method of using the same, which includes a remotely placed monitor station in communication with a diaper device. The diaper device has electronic components that are configured to detect an increase in conductivity across opposing detector electrodes, which increase can then be used to indicate, via an EMF signal, that the diaper device is wet. The electronic components of the remotely placed monitor station are configured to receive the EMF signal and to alert the health care worker that the diaper device is in need of being changed.

Such a system may not be able to cost-effectively differentiate between the passing of a volume of urine which can be completely absorbed by the diaper, and saturation or fouling by excrement of a diaper.

### Summary

According to a first aspect of there is disclosed a soiling alert transmitter for integration into an incontinence garment having an inward-facing surface region, the transmitter comprising: a sensor configured to sense humidity changes in the inward-facing surface region of the incontinence garment; a processing unit configured to detect whether a sensed change is a transient change; and a wireless transmitter, which may short-range and in particular may be a magnetic induction radio, configured to transmit an alert signal in response to the detection of a change which is not a transient change.

By differentiating whether the sensed change is a transient change or a permanent change, it may thus be possible to differentiate between, in the first case urination which does not saturate the incontinence garment, and in the second case either urination which does saturates the incontinence garment or excretion which fouled the incontinence garment. It will be appreciated that as used herein, the term "surface region" refers to a three-dimensional volume rather than to a planar, 2-dimensional, surface, and soiling may refer to excretion into the incontinence garment or urination resulting in saturation.

As used herein, the term" transient" indicates a period of time which is sufficiently short such that the risk of skin irritation due to prolonged wetness is reduced, mitigated or avoided. Such transients will typically be limited to a minute or less - but may be shorter, such as less than 15 seconds. A change in a parameter may be considered to be not transient, if the parameter does not return to within a predetermined percentage, such as 5%, 20% or 50%, of its original value, with the above mentioned period. The inward-facing surface region may face an interior region.

In embodiments the sensor comprises a pair of electrodes configured to extend over at least one quarter of the inward-facing surface region of the incontinence garment and the sensor is configured to sense humidity changes by detecting changes in at least one of conductance and capacitance between the pair of electrodes. By extending over at least one quarter of the inward-facing surface region of the incontinence garments, the electrodes may be able to detect a urination or fouling event which need not be localised to the integrated circuit itself. It will be noted that the sensed area may be different for incontinence garments designed specifically for female wears and incontinence garments designed specifically for male wearers. In particular, for females, the electrodes may extend across a relatively smaller area, in comparison with those in an incontinence garment designed for male wearers.

In some embodiments the pair of electrodes comprises an arrangement of interdigitated fingers; in some embodiments, the pair of electrodes comprises a meandering pair of adjacent parallel tracks; and in some embodiments the electrodes are arranged in separate planes. It will be appreciated that many different geometrical arrangements for the electrodes are possible and the above examples are not limiting, and may be combined, at least partially.

According to another aspect there is provided an incontinence garment comprising a soiling alert transmitter as described above. The soiling alert transmitter may be integrated into the incontinence garment during the latter's manufacture, thereby providing opportunity for cost saving, relative to the subsequent, that is to say, post-manufacturing, add-on or introduction of the soiling alert transmitter into a standard incontinence garment. The incontinence garment may be a diaper.

According to a yet further aspect there is provided a soiling alert system, comprising a transmitter as just described, and a radio receiver which may be a magnetic induction radio receiver. In embodiments the receiver is configured to be worn on the body of a patient wearing the incontinence garment. In embodiments, the receiver further comprises a wireless transmitter adapted to, in response to receiving an alert signal, transmit a soiling alert to a patient-care system. By separating the receiver, which may forward the alert signal as a soiling alert to a remote network, from the radio transmitter which may be a magnetic induction radio transmitter, the latter may require relatively less power, since the transmitter is only required to transmit information to a nearby object - the body worn receiver. The space requirements for the soiling alert transmitter may thereby be kept low, and the the inconvenience to the wearer be kept to a minimum; in general wearing a bracelet may be less disruptive to the wearer, than wearing an incontinence garment with a relatively large soiling alert transmitter integrated therein.

According to a yet further aspect, there is provided a soiling alert system, comprising a transmitter and a receiver; the transmitter being for integration into an incontinence garment having an interior region and an inward-facing surface region, wherein the transmitter comprises: a sensor configured to sense humidity changes in the inward-facing surface region of the incontinence garment; and a wireless transmitter configured to transmit change signals in response to the detection of sensed changes; and wherein the receiver comprises a processing unit configured to detect whether the sensed changes result from a transient change and to create a soiling alert signal in response to detecting that a change is not a transient change. In embodiments, the wireless transmitter may be a magnetic induction radio; in other embodiments, the wireless transmitter may be based on, for instance, Wifi, Zigbee, Bluetooth or other protocols and in particular protocols or hardware adapted for short-range transmission.

According to this aspect, the determination of whether a change is transient, and thus the diaper is not saturated or otherwise soiled, is made not within the diaper itself, but in the receiver, which may generally be a body worn receiver, such as a wristband unit; this may reduce the requirement for processing power, and thus size, of the transmitter unit within in the diaper; conversely, more energy may be required to transmit signals to the receiver for processing than would be required in the case that the processing unit is comprised in the transmitter.

According to a yet further aspect, there is provided a method of detecting soiling of an incontinence garment, the method comprising: sensing a change in either conductance or capacitance over the surface of the garment, differentiating between a non-transient and a transient change in conductance or capacitance, and in response to a non-transient change, transmitting a signal indicative of a soiled condition of the incontinence garment.

These and other aspects of the invention will be apparent from, and elucidated with reference to, the embodiments described hereinafter.

### Brief description of Drawings

Embodiments of the invention will be described, by way of example only, with reference to the drawings, in which
figure 1 shows schematically a cross-section through a diaper according to embodiments;
figure 2 shows in outline a plan view of the diaper of figure 1;
figure 3 shows the change in humidity over time in a surface region of a diaper, on the incidence of urine which is insufficient to saturate the diaper;
figure 4 shows the change in humidity over time in a surface region of the diaper on the incidence of urine which saturates the diaper;
figure 5 shows the change of humidity over time in a surface region of the diaper on the occurrence of fouling of the diaper by excrement - a stool or faece;
figure 6 shows an arrangement of electrodes according to embodiments;
figure 7 shows an alternative arrangement of electrodes according to environments;
figure 8 shows a further arrangement of electrodes according to embodiments;
figure 9 shows a soiling alert system according to embodiments;
figure 10 shows a system according to embodiments;
and figure 11 shows a different system according to other embodiments.

It should be noted that the figures are diagrammatic and not drawn to scale. Relative dimensions and proportions of parts of these Figures have been shown exaggerated or reduced in size, for the sake of clarity and convenience in the drawings. The same reference signs are generally used to refer to corresponding or similar feature in modified and different embodiments

### Detailed description of embodiments

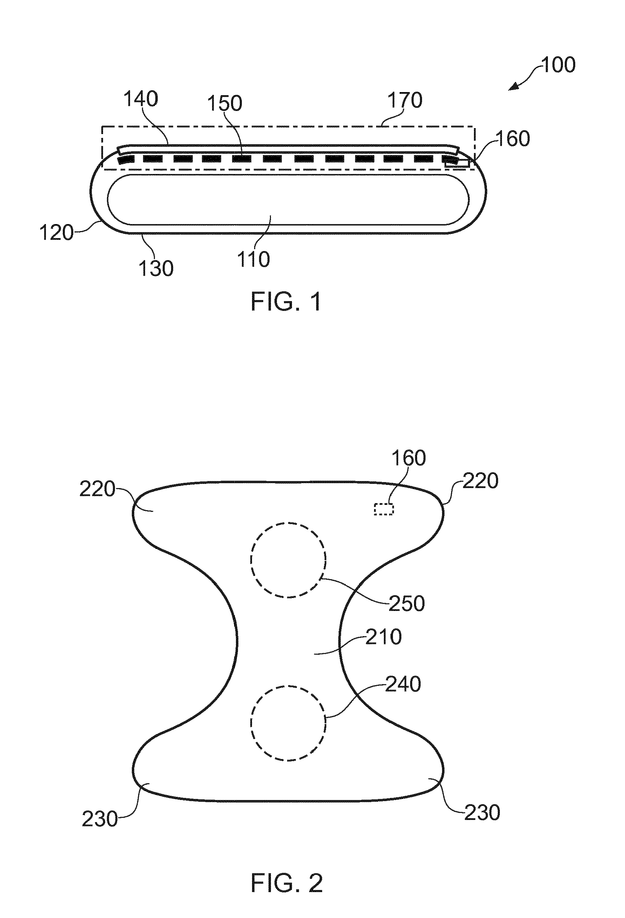

Figure 1 shows schematically a cross-section through a diaper 100 according to embodiments. Most of the volume of the diaper 100 comprises paper pulp 110, inside the lower part of which is a hygroscopic absorbing material which sucks fluids into it. The paper pulp volume 110 is retained in the diaper by a wrapper 120. On the face 130 of the diaper which is outward facing with respect to the wearer, the material of the wrapper is chosen for aesthetic or practical reasons. On the face of the diaper which is intended to be in contact with the skin of a wearer, the wrapper is a skin contact layer 140, the material for which is selected such that it has a "feel-dry" feeling, and generally avoids the incidence of skin irritation or allergies in humans.

Adjacent to the skin contact layer 140, and in the inward-facing surface region 170 of the diaper which is intended to be placed against to the wearer's skin, is included a network of electrodes shown at 150. These electrodes will be described in more detail hereinelow. In electrical contact with the electrodes 150 is an integrated circuit 160. Integrated circuit 160 may comprise a processor unit and a wireless transmitter. Although there is no limitation thereto, in specific embodiments the wireless transmitter is a magnetic induction (MI) radio transmitter, since MI radios may be particularly convenient, small and inexpensive. In other embodiments, the wireless transmitter may be based on, for instance, Wifi, Zigbee, or other protocols and in particular protocols or hardware adapted for short-range radio transmission.

As will be explained in more detailed hereinbelow, the processing unit is configured to distinguish between a transient change (in either conductance or capacitance), and a permanent change.

However, in other embodiments, the integrated circuit does not include this processing functionality; in these embodiments, a processing unit is included in the receiver which is adapted to receive the transmission from the wireless transmitter. Due to the typically short range of the transmission, the receiver will typically be worn on the body of a user, and in these embodiments, the determination of whether the diaper has been soiled (or is saturated) and thus needs to replacing, that is to say, the creation of the soiling alert information, may be carried out by the processing unit located in the receiver. In such embodiments, the transmitter may transmit signals corresponding to changes in conductance or capacitance, over a period of time. The processing unit may then analyse the changes, and in particular may analyse whether the conductance, or capacitance as the case may be, returns to within a predetermined closeness to its original level within a predetermined time, in order to establish whether the changes are a transient or not. The skilled person will appreciate that, in order not to overload the wireless transmissions, a threshold may be set such that the transmitter only transmits a change which is greater than a predetermined threshold.

It will be appreciated, that in both cases, the soiling alert is generated localised to the user. Extensive communication with a remote monitoring station or other monitoring system may thus not be required.

Figure 2 shows the general outline of a diaper according to embodiments, in plan view. The diaper has a relatively narrow central section 210 which is designed to be wrapped between the wearer's legs, and has wings 220 and 230 at respective ends for positioning at the wearer's front and back, to enable the ends of the diaper to be fastened together, for instance by adhesive, Velcro, or other means as will be familiar to the skilled person. It will be immediately apparent to the skilled person that there is no single position in the diaper at which it might be expected to be both fouled by the excretion of a stool or faeces, or wetted by the passing of urine, since the former typically occurs at the anus, towards the rear of the diaper, in the region generally shown at 250, and the latter typically at the urethra, towards the front of the diaper, in the region generally shown at 250.

Although the integrated circuit 160 is not visible in the plan view shown in this figure, an example of its location is shown dotted. Such a position, in a wing at the front of the diaper, is convenient in that it provides a relatively short path for signals which may typically be transmitted to a wearer's wrist. However, it will be appreciated that, in other embodiments, the integrated circuit may be located elsewhere.

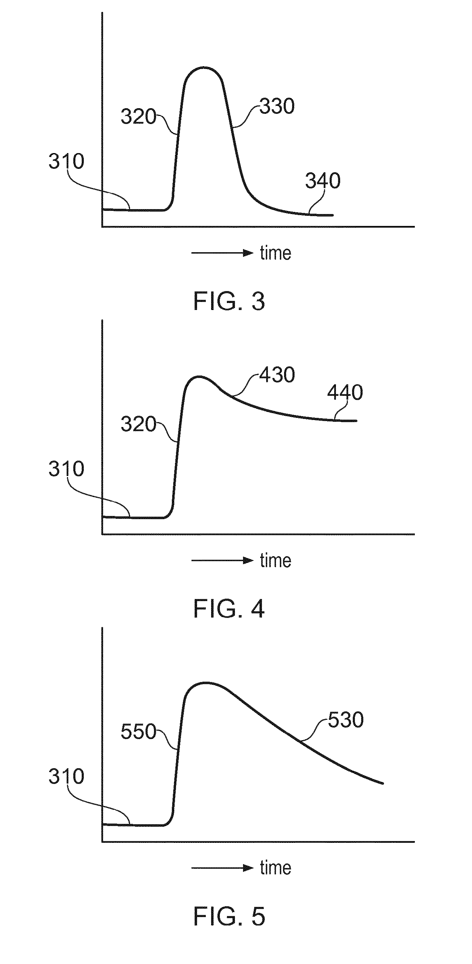

According to embodiments, a change in humidity within the inward-facing surface region, which results from the passing of urine or excretion of a stool, can result in a change in conductance or capacitance between electrodes located within that surface region. The electrodes are connected to a processing unit that detects such a change in conductance or capacitance and thus monitors the humidity of the inward-facing surface region of the diaper. When the wearer passes urine, there will be an increase in humidity of this inward-facing surface region. The hygroscopic material in the bulk will attract moisture away from the inward-facing surface region, in a way which is familiar to those skilled in the art, in order to maintain the "keep dry" feel of the diaper. Thus, provided that the diaper is not saturated, the humidity of the inward-facing surface region of the diaper will reduce after the passing of the urine back towards its previous level. Thus there is a transient - which may be relatively short - in the humidity of the inward-facing surface region. This is shown in figure 3, in which the humidity at the inward-facing surface region of the diaper is plotted on the Y-axis or ordinate against time on the X-axis or abscissa. Initially the humidity has a low value shown at 310, corresponding to the keep-dry feel of the diaper. Upon the passing of urine, there is a sharp increase in humidity as shown at 320; however due to the hygroscopic effect of the absorbent material, this transient is short-lived, and the relative humidity falls, as shown at 330, back generally to, or nearly to, its original value at 340.

In contrast, if the diaper is saturated, the hygroscopic material in the pulp is unable to efficiently attract moisture away from the inward-facing surface region. This situation is shown in figure 4, in which the humidity of the inward-facing surface region 170 is plotted against time. In this case, the relative humidity of the inward-facing surface region starts at a low value 310, and sharply increases when the urine is passed, as shown at 320. However, in this case the humidity is unable to fall quickly, as shown at 430, but only partially returns, and settles at a much higher value than its original value, as shown at 440.

A further situation is shown in figure 5: once more this figure shows the humidity of the inward-facing surface region 170 plotted against time, but this time the event is the excretion of a stool. Once again, the humidity starts at a relatively low level, and increases rapidly when the excretion occurs. It will be noted that, although as shown in figure the increase in humidity may occur at a similar speeds as for the passing of urine, in some embodiments the increase may be somewhat slower, due to the relatively low high viscosity of the stool material relative to urine. Similar to the situation in passing urine, the humidity peaks, and then starts to fall as the hygroscopic material in the bulk of the diaper attracts moisture away from the inward-facing surface region 170. However, due to the solids content of the material in a stool, moisture is retained at the surface of the diaper for a longer period, and so the decrease in humidity is significantly slower than is the case for the passing of urine (provided that the diaper is unsaturated).

It is thus possible to differentiate distinguish between two cases: in the first case, urine is passed but the diaper is not saturated. There is an increase in humidity, but only for a relatively short transient. It is not necessary to change the diaper. In the second case, either urine is passed and the diaper is saturated, or a stool is excreted: in either event, the increase in humidity is significantly more long-lasting, and is either permanent or semi-permanent. It is necessary to change the diaper. Thus by distinguishing between a transient increase in humidity, and one which is either permanent or semi-permanent, it is possible to determine whether or not it is necessary to change the diaper.

In a typical but non-limiting experimental arrangement, it has been found that in the case of urine and a diaper with a "feel dry" construction, the transient is particularly short: the conductance may peak at over 10 times its original level, but within 10 seconds, it has reduced to within 10% of its original value. It will be appreciated that the speed with which the transient disappears will be, to a limited extent, a function of the construction of the specific diaper.

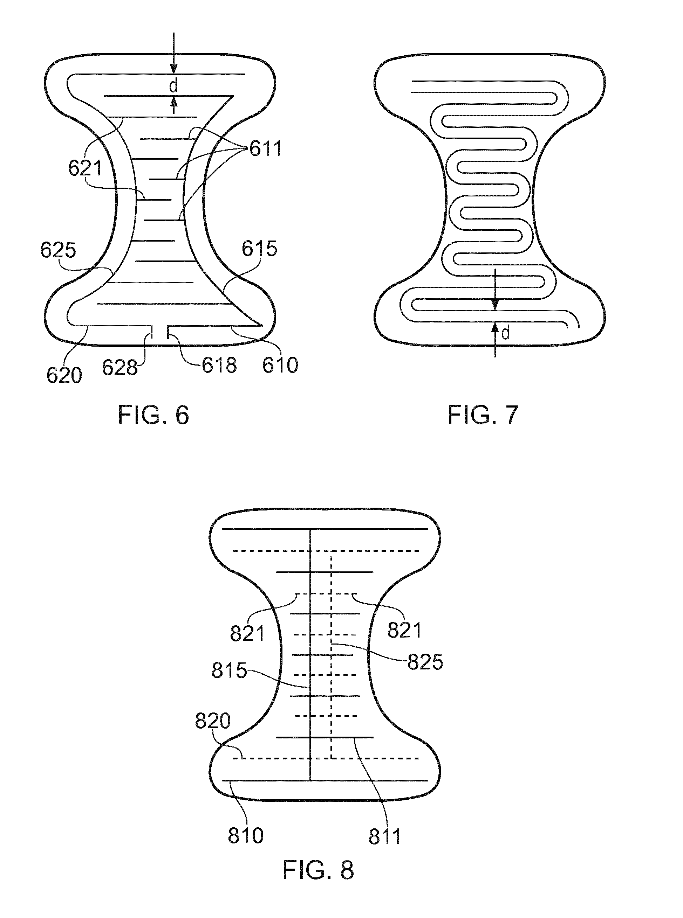

In order to accurately determine the humidity in the inward-facing surface region, it is preferred that the humidity sensor is not localised, but is distributed - that is to say it extends over a significant part of the surface area. This may be achieved by using a pair of electrodes arranged in a network configuration. Examples of such networks are shown in figures 6, 7 and 8.

The sensor is based on a pair of electrodes, and either the conductance between the electrodes or the capacitance between electrodes, or both, is measured: a change in humidity in the region between electrodes results in a change in the effective dielectric constant between the electrodes. Thus a capacitor formed by the two electrodes and having the region between as dielectric material will show a change in capacitance upon a change in humidity. Also, a change in humidity of the region between the two electrodes will result in a change in resistance of that region, which may be measured by sensing the conductance between the electrodes.

Figure 6 shows a first arrangement of electrodes according to embodiments. In this arrangement, two electrodes 610 and 620 each comprise a central spine 615 and 625 respectively, attached to which is a plurality of fingers respectively 611 and 621. Each electrode spine (615, 625) is arranged generally along one respective side of the diaper, and the fingers 611 and 621 are inter-digitated, to provide a generally consistent gap 'd' between the two electrodes 610, 620. As shown, electrode contacts 618, 628 may be positioned centrally along the back or bottom face of the diaper. The skilled person will appreciate that the contact may be positioned differently, for instance in one of the wings of the diaper, in order to correspond to a convenient position of the integrated circuit (not shown in this figure).

Since the electrodes are "distributed" in that they extend across a large part of the surface area of the diaper, it may be possible to sense a change in one or both of conductance and capacitance, resulting from a change in humidity, wherever it occurs across that large part of the diaper. In general, the electrodes may be able to detect a change in humidity in any part of the diaper over which they extend. Since soiling and wetting, and thus changes in humidity, are not expected to occur over some parts of the diaper (for instance at the extremities of the wings), it may not be required to extend the electrodes over these parts of the diaper. However, in general it will be expected that ensuring the electrodes are distributed over at least one quarter of the surface area of the diaper, and typically over one half or three quarters may be desirable in order to sense, with a reasonable degree of certainty, any event of passing of urine or excretion of a stool.

Figure 7 shows an alternative arrangement of electrodes according to embodiments; once again, the pair of electrodes are distributed in the sense that they extend over a significant part of the surface area of the diaper. In this case, the electrodes comprise a meandering pair of adjacent, generally parallel, tracks. That is to say, the tracks are generally parallel in the sense that there is generally a common and uniform distance between the two electrodes all the way along the track. However, in order to extend across a significant part of the surface area of the diaper, the parallel tracks are made to meander. In the example shown, the meanders take the form of a series of parallel straight paths, with 180° turns between adjacent parts. However, the skilled person will appreciate that other forms of meander may be used in addition or alternatively; for instance the parallel tracks may be arranged to follow the contours of the perimeter of the diaper and spiral in towards the centre.

In each of the arrangement shown in figure 6 and 7, the electrodes may be provided by being printed onto a single surface of one layer in a multilayer stack or laminate which forms the outward facing surface region of the diaper. As a non-limiting alternative, the tracks may be provided by being woven into a material which forms a layer of a multilayer stack or laminate. As a further non-limiting alternative, the electrodes may be deposited conductive material, rather than printed or woven material.

In each of these non-limiting examples, the electrodes may be considered to be planar, since the separation is generally in the same plane and the electrodes are on or in the same layer of material. In other embodiments, the electrodes may be non-planar or multi-planar, or may be arranged in different planes. A non-limiting example of such a pair of electrodes is shown in figure 8. Figure 8 shows schematically a first electrode 810 arranged with fingers 811 extending in both directions from a central spine, or bus, 815. This electrode is, in this example, arranged in a single plane, which may be one surface of a layer of a laminate or stacked structure. In a separate plane, which may be the opposite surface of the same layer, or may be a surface of another layer in a laminate or stacked structure, is arranged the other electrode 820. In this example the second electrode 820 is also arranged as a central spine, or bus, 825, extending from which in both directions are fingers 821. The construction - that is so say the materials or dimensions - of the central spine or bus, 815, 825, of each electrode may be the same as, or different from, the fingers 820, 821. In particular in the the case that they are different, it may be possible to determine spatial gradients over the diaper surface.

The skilled person will appreciate that there are many alternative arrangements of the electrodes, which in general have a common or uniform separation between them. Providing a common or uniform separation may result in a consistent sensitivity of the sensor to changes in humidity which might occur in different positions or locations in the diaper. That is to say, the same change in humidity may be expected to result in the same change of either conductance or capacitance irrespective on the location on the diaper, if the separation between the two electrodes is the same irrespective of the location on the diaper. In other embodiments it may be preferred to provide a separation between the electrodes which is not uniform across the whole diaper. The same change in humidity may then result in a change in conductance or capacitance which is dependent on and varies with the location on the diaper where the changes occurs - if localised. If the level of the change in humidity is known - for instance if it may be expected to reach close to 100% - then this variation may be used to indicate where, on the diaper surface, the change has occurred.

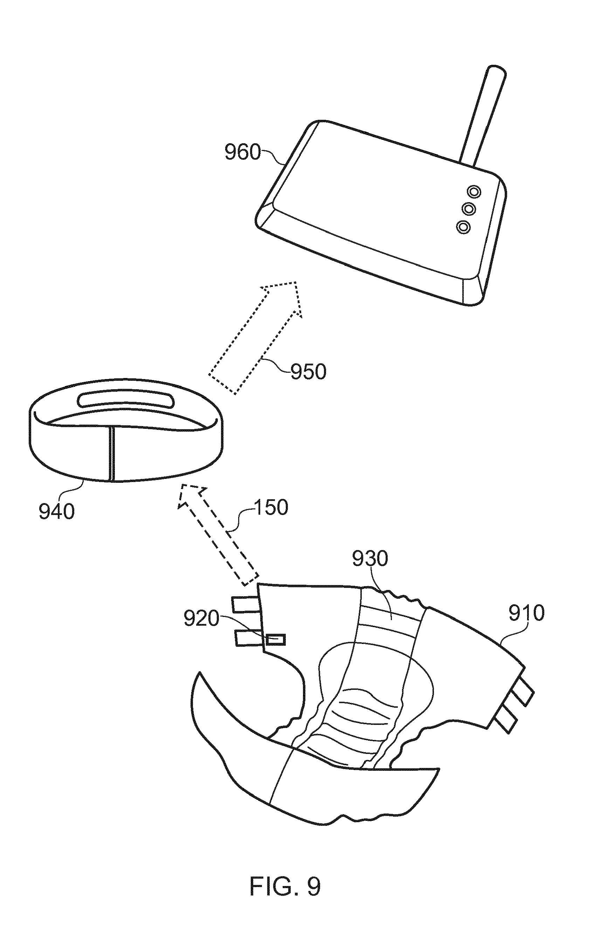

Figure 9 shows a soiling alert system according to embodiments. The system comprises an incontinence garment such as diaper 910, integrated into which is a soiling alert transmitter 920, having a pair of distributed electrodes extending across at least part of an inward-facing surface 930 of the incontinence garment. The soiling alert transmitter 910 comprises a magnetic induction radio which is configured to communicate with a receiver 940.

Magnetic induction radio transmission is generally short range in nature, the signal strength falling off rapidly with distance from the transmitter, according to at least the inverse cube of the distance, in contrast to conventional far field electromagnetic wave radio transmission, in which the signal strength falls with the inverse square of the distance.

The receiver 940 should generally be located nearby the transmitter, and typically will be worn on the body of the wearer of the incontinence garment. Without limitation, the receiver may be worn as an anklet or as a necklace or, as shown in figure 9, as a bracelet. The receiver 940 is configured to receive an alert signal 150 from the magnetic induction radio. The receiver is further configured to forward the alert signal to a patient care system. Before forwarding transmission of the alert signal, the receiver 940 may add related information such as the patient's identity, a timestamp, and the like, to the alert signal, to create a soiling alert which is specific to the patient. The additional information may be pre-programmed into the receiver 940. The transmission of the soiling alert that 950 may be by means of known protocols, such as for instance using the Zigbee or Bluetooth transmission protocols, or may use another or proprietary transmission protocol. The transmission is received by a base unit 960, which may form part of an integrated patient care system.

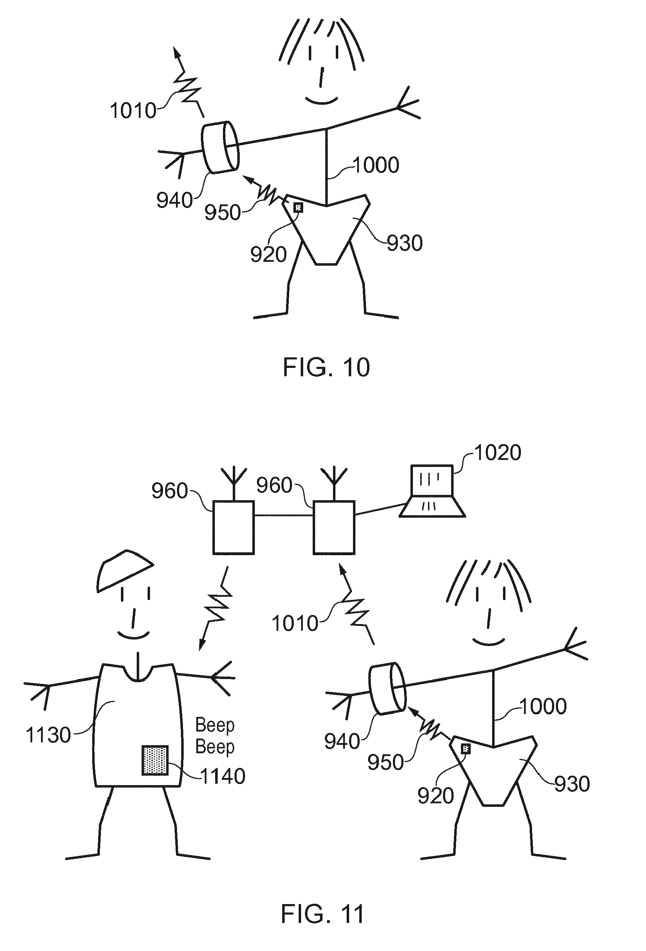

Figure 10 shows a system according to embodiments, in which there is not an integrated patient care system. In such a system, when a soiling event occurs such that a patient 1000 fouls a diaper 930 or urinates such that the diaper is saturated, magnetic induction radio 920 transmits alert signal 950 to a receiver 940 shown as a bracelet. Receiver 940 adds patient identification information to the alert signal 950 and transmits a soiling alert signal 1010 to a carer. In the example of figure 10, the carer may a single carer, who directly receives the soiling alert signal 1010 from the patient's receiver 940, and is thus alerted to provide patient care by changing the incontinence garment.

Figure 11 shows a different system according to other embodiments, which system is designed for use in an integrated, or extended, patient care system. The system is generally similar to that shown in figure 10 wherein the patient 1000 wears a diaper 930. Upon soiling or saturation of the diaper, magnetic induction radio 920 transmits a signal 950 to the patient's receiver 940, which is shown as a bracelet. The receiver 940 may add patient identification information and other relevant information, and forwards the alert signal as a soiling alert 1010 to a base unit 960. The base unit is in communication with a computer network 1120. Network 1120 may include additional base units 960. Upon receipt of a soiling alert, network 1120 may broadcast the soiling alert to all base units 960. One or more healthcare workers 1130 may carry or wear an alarm or "bleep" 1140, which may be configured to react to a broadcast sliding alert. The alarm or bleep 1140 may be configured to react to all broadcast soiling alerts, or only to a subset of broadcast soiling alerts which relate to specific patients. Reaction of the alarm or bleep 1140 provides notification to the healthcare worker to change the incontinence garment. The alarm or bleep 1140 may be a standalone dedicated unit, or may be functionality integrated into another device - such as a, for instance, a mobile phone, so-called "smart phone", or pager.

The computer network may be programmed to provide additional functionality: for instance in the absence of any alert signal from a specific patient for a prolonged period of time, a healthcare worker may be alerted to investigate in case there is a malfunction of the receiver 940, or of a soiling alert transmitter in a incontinence garment being worn by that patient. Further, the computer network may provide inventory control by keeping track of how many incontinence garments have been used over a particular period. The computer network may even provide billing information, by recording the frequency of changing of any specific patient, thereby identifying a level of sanitary care required by that patient.

The above embodiments have been described with reference to diapers as the incontinence garments. It will be appreciated that other incontinence garments such as sanitary pads may be used instead of diapers. Further, it will be appreciated, that due in part to the relatively short- range transmission of the magnetic induction radio, a diaper or other incontinence garment may transmit its alert signal only to a nearby receiver, which will in general be that worn by the patient themselves. Identification information as to which patient requires care may thus not need to be embedded in the alert signal from the diaper to the receiver, since this information can be added by the receiver before forwarding the alert as a soiling alert. In this way, the diapers used on any individual patient may not have to be pre-programmed or prepared in any specific fashion, which may keep the complexity of the system is low. To avoid "false-positive" signals, for instance where two patients are in close contact such that the receiver being worn by a first patient may receive a transmission from a second patient's diaper, the diaper may transmit a identification signal to the receiver, for instance when it is first applied to the patient, in order to avoid later "false-positive" signals. In other embodiments, the diaper and receiver may undergo a "pairing" arrangement upon the diaper being applied.

From reading the present disclosure, other variations and modifications will be apparent to the skilled person. Such variations and modifications may involve equivalent and other features which are already known in the art of smart incontinence garments, and which may be used instead of, or in addition to, features already described herein.

Although the appended claims are directed to particular combinations of features, it should be understood that the scope of the disclosure of the present invention also includes any novel feature or any novel combination of features disclosed herein either explicitly or implicitly or any generalisation thereof, whether or not it relates to the same invention as presently claimed in any claim and whether or not it mitigates any or all of the same technical problems as does the present invention.

Features which are described in the context of separate embodiments may also be provided in combination in a single embodiment. Conversely, various features which are, for brevity, described in the context of a single embodiment, may also be provided separately or in any suitable sub-combination. The applicant hereby gives notice that new claims may be formulated to such features and/or combinations of such features during the prosecution of the present application or of any further application derived therefrom.

For the sake of completeness it is also stated that the term "comprising" does not exclude other elements or steps, the term "a" or "an" does not exclude a plurality, a single processor or other unit may fulfil the functions of several means recited in the claims and reference signs in the claims shall not be construed as limiting the scope of the claims.

## Claims

1. A soiling alert transmitter for integration into an incontinence garment having an inward-facing surface region, the transmitter comprising:
a sensor configured to sense humidity changes in the inward-facing surface region of the incontinence garment;
a processing unit configured to detect whether a sensed change is a transient change; and
a wireless transmitter configured to transmit an alert signal in response to the detection of a change which is not a transient change.

2. A soiling alert transmitter according to claim 1, wherein the wireless transmitter is a magnetic induction radio.

3. A soiling alert transmitted according to claim 1 or 2, wherein the processing unit is configured to determine that a sensed change is a transient change in the case that the humidity returns to within 25% of its original value within 1 minute.

4. A soiling alert transmitter according to any preceding claim, wherein the sensor comprises a pair of electrodes configured to extend over at least one quarter of the inward-facing surface region of the incontinence garment and the.sensor is configured to sense humidity changes by detecting changes in at least one of conductance and capacitance between the pair of electrodes.

5. A soiling alert transmitter according to claim 4, wherein the pair of electrodes comprises an arrangement of interdigitated fingers.

6. A soiling alert transmitter according to claim 4 or 5, wherein the pair of electrodes comprises a meandering pair of adjacent parallel tracks.

7. A soiling alert transmitter according to any of claims 4 to 6, wherein the electrodes are arranged in separate planes.

8. An incontinence garment comprising a soiling alert transmitter according to any preceding claim.

9. A soiling alert system, comprising a transmitter as claimed in any preceding claim, and a receiver.

10. A soiling alert system as claimed in claim 9, wherein the receiver comprises a magnetic induction radio receiver.

11. A soiling alert system as claimed in claim 9 or 10, wherein the receiver is configured to be worn on the body of a patient wearing the incontinence garment.

12. A soiling alert system, comprising a transmitter and a receiver;
the transmitter being for integration into an incontinence garment having an interior region and an inward-facing surface region, wherein the transmitter comprises:
a sensor configured to sense humidity changes in the inward-facing surface region of the incontinence garment; and
a wireless transmitter configured to transmit change signals in response to the detection of sensed changes;
and wherein the receiver comprises a processing unit configured to detect whether the sensed changes result from a transient change and
to create a soiling alert signal in response to detecting that a change is not a transient change.

13. A soiling alert system according to claim 12, wherein the wireless transmitter is a magnetic induction radio transmitter and the receiver comprises a magnetic induction radio receiver.

14. A soiling alert system as claimed in any of claims 10 to 13, wherein the receiver further comprises a wireless transmitter adapted to, in response to receiving an alert signal, transmitting a soiling alert to a patient-care system.

15. A method of detecting soiling of a incontinence garment, the method comprising:
sensing a change in either conductance or capacitance over the surface of the garment,
differentiating between a non-transient and a transient change in conductance or capacitance, and in response to a non-transient change,
transmitting a signal indicative of a soiled condition of the incontinence garment.
